(19) Europäisches Patentamt
European Patent Office
Office européen des brevets

(11) Publication number: **0 165 636**
**B1**

# (12) EUROPEAN PATENT SPECIFICATION

(45) Date of publication of patent specification: **10.08.88**

(51) Int. Cl.[4]: **C 07 D 205/04**

(21) Application number: **85200881.2**

(22) Date of filing: **04.06.85**

## (54) Process for preparing azetidine derivatives and intermediates therein.

(30) Priority: **19.06.84 GB 8415615**

(43) Date of publication of application:
**27.12.85 Bulletin 85/52**

(45) Publication of the grant of the patent:
**10.08.88 Bulletin 88/32**

(84) Designated Contracting States:
**AT BE CH DE FR GB IT LI LU NL SE**

(56) References cited:
**FR-A-1 479 735**

**TETRAHEDRON, vol. 32, no. 22, 1976, pages 2689-2692, Pergamon Press, GB; R.H. FISH et al.: "Gas phase protonolysis reactions chemical ionization mass spectrometry of N-nitrosamines"**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **De Nie-Sarink, Margaretha Johanna**
**Badhuisweg 3**
**NL-1031 CM Amsterdam (NL)**
Inventor: **Mason, Ronald Frank**
**"Kingswood" Westwell**
**Ashford Kent (GB)**

(74) Representative: **Hunter, Keith Roger Ian et al**
**4 York Road**
**London SE1 7NA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 165 636 B1

**Description**

This invention relates to a process for preparing azetidine derivatives, more specifically azetidine-3-carboxylic acid and salts thereof, and to certain intermediates in the process.

European Patent Application Publication No. 29265 discloses that 3-carboxyazetidine (azetidine-3-carboxylic acid) and related compounds are chemical hybridising agents, their mode of action presumably being based on their ability to produce male sterility in plants. That application also describes a process for their preparation, starting from 3-cyano-1-diphenylmethylazetidine, which may be prepared by methods known *per se*. Although the process described works well, it is not ideally suited for large scale preparations, since the bulky diphenylmethyl group on the nitrogen atom is removed only in the last of a series of steps, which means that in all but the last step comparatively large equipment is needed relative to the weight of the final product. Moreover, the apparent starting compound (diphenylmethyl)amine is relatively expensive.

It has now surprisingly been found that azetidine-3-carboxylic acid and salts thereof may be prepared by a process involving a reaction step in which a 3,3-bis(hydroxymethyl)-azetidine is treated with nitric acid.

According to the present invention there is provided a process for preparing azetidine-3-carboxylic acid

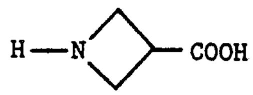

(I)

or a salt thereof, which comprises treating a compound of formula

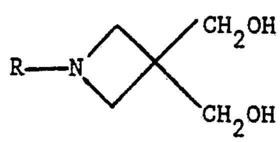

(II)

wherein R represents a hydrogen atom or a group of formula $R^1R^2CH—$ wherein $R^1$ and $R^2$ are independently selected from hydrogen, alkyl (preferably $C_{1-6}$alkyl) and optionally substituted aryl (preferably $C_{6-12}$aryl) moieties, with nitric acid and subjecting the resulting product to acid conditions at elevated temperature to produce an acid-addition salt of formula I, and thereafter, if desired, converting the acid addition salt to the free acid or another salt of the acid of formula I.

Optional substituents in an aryl moiety include one or more substituents selected from the group consisting of $C_{1-4}$alkyl, $C_{1-4}$alkoxy, halo, nitro and ($C_{1-6}$alkoxy)carbonyl moieties.

Preferably at least one of $R^1$ and $R^2$ in the group $R^1R^2CH—$ is hydrogen. Conveniently R in formula II represents a hydrogen atom or a group of formula $R^1CH_2—$ wherein $R^1$ represents hydrogen, a $C_{1-3}$alkyl group or a phenyl group.

The compound of formula II may conveniently be treated with nitric acid at a temperature in the range 35°C to the reflux temperature of the reaction mixture, conveniently a temperature in the range 40 to 85°C. Temperatures in the range of 45 to 60°C are very effective.

It is preferred that the concentration of nitric acid used in the treatment of the compound of formula II be at least 40%w/w. Conveniently the concentration of the nitric acid used is in the range 50 to 65%w/w.

Those skilled in the art will appreciate that the resulting product may, or may not, be isolated prior to the step of subjecting it to acid conditions.

Preferably the step of subjecting the resulting product to acid conditions comprises treating the product with an aqueous acid selected from nitric, formic, acetic, hydrohalic (e.g. hydrochloric) and sulphuric acids. If nitric acid is used, its concentration is preferably 30%w/w or less.

The resulting product is preferably subjected to acid treatment at temperatures above 80°C, more preferably above 90°C. Conveniently the resulting product is subjected to acid conditions at the reflux temperature of the reaction mixture.

Those skilled in the art will appreciate that in acid conditions the compound of formula I will generally exist as an acid-addition salt. If the acid is formic acid, it may be driven off with water when an aqueous solution is evaporated to dryness, to liberate the free acid. In other cases, acid-addition salts can readily be converted to the free acid, or to other acid-addition salts by conventional known methods.

The preparation of some compounds of formula II is described in UK Patent Specification No. 1,169,027 and other compounds of formula II may be prepared by analogous methods. Alternatively, compounds of formula II may be prepared by processes described hereinafter in detail, or by obvious modification of such processes.

The invention also comprehends azetidine-3-carboxylic acid or a salt thereof whenever prepared by the process of the invention.

Intermediates which may be produced in the process of the invention include azetidine-3,3-dicarboxylic acid, 1-nitroso-azetidine-3,3-dicarboxylic acid and 1-nitrosoazetidine-3-carboxylic acid. *Inter*

*alia,* azetidine-3,3-dicarboxylic acid is subject of Applicants' copending European patent application No. 85200882.0 (Publication No. 165637). The present invention includes as novel compounds the 1-nitroso-azetidine-3-carboxylic acid derivatives of formula

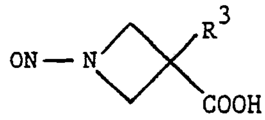

(III)

or salts thereof, wherein $R^3$ is hydrogen or a group of formula COOH.

The invention will be further understood from the following examples, of which Examples 1 to 7 illustrate the preparation of starting materials and Examples 8 to 19 illustrate processes in accordance with the invention.

Example 1

Preparation of 3,3-bis(hydroxymethyl)-1-benzylazetidine

(i) 2,2-Bis(bromomethyl)-1,3-propanediol (60.0 g; 22.9 mmol) was dissolved in ethanol (200 ml) and NaOH (10.1 g, 98% pure; 24.7 mmol) was added. The reaction mixture was stirred for 3 hours at reflux temperature. Subsequently the solvent was evaporated off at reduced pressure, the residue was taken up in diethyl ether and the solid material (sodium bromide) was removed by filtration. The resulting ethereal solution was evaporated to dryness. The residue was distilled at reduced pressure to yield 3-bromomethyl-3-hydroxymethyl oxetane (40.0 g, purity 94%, isolated yield 66.4% on intake 2,2-bis(bromomethyl)-1,3-propanediol as a clear, colourless oil. (Bp. 101—110°C at 133 Pa (1 mm Hg)).

(ii) 3-bromomethyl-3-hydroxymethyl oxetane (18.1 g; 100 mmol) and benzylamine (11.7 g; 110 mmol) were taken up in water (5 ml) and the reaction mixture was stirred at 100°C. After 4 hours the solution was allowed to cool to ambient temperature (20°C) and 36% hydrochloric acid (w/w) (13.8 ml; 1.46 eq. on benzylamine) was added. The resulting mixture was stirred at 60°C. After 2 hours the solution was again allowed to cool to room temperature and was subsequently washed with dichloromethane (50 ml) to remove by-products. The aqueous layer was made alkaline by the addition of sodium hydroxide (5.5 g) in water (10 ml) and stirred at 100°C for 1 hour. After cooling to ambient tmeperature the pH of the mixture was raised by addition of another 5.5 g sodium hydroxide in 10 ml water and the mixture washed with hexane to remove benzylamine. The aqueous layer was concentrated by evaporation of the water under reduced pressure, and the solid residue was taken up in dichloromethane. Inorganic salts, which remained undissolved, were removed by filtration, and the dichloromethane was evaporated in vacuo, leaving 3,3-bis(hydroxymethyl)-1-benzylazetidine as a white crystalline solid. The isolated yield after recrystallisating twice from toluene was 80% on the starting oxetane.

Example 2

Preparation of 3,3-bis(hydroxymethyl)azetidine

3-bromomethyl-3-hydroxymethyl oxetane (7.5 g; 41 mmol) was taken up in 25% (w/w) aqueous ammonia (100 ml) and the mixture was stirred for 16 hours in an autoclave at 80°C. After evaporation of the aqueous ammonia, 48% (w/w) aqueous hydrobromic acid (10 ml) was added to the residue and the resulting solution was stirred at 60°C. After 2 hours the aqueous hydrobromic acid was evaporated off, 25% (w/w) aqueous ammonia (75 ml) was added to the residue, and the resulting solution was stirred at 60°C. After 2 hours the aqueous ammonia was evaporated. The white, solid residue was freed from ammonium bromide by purification over "Dowex" 50W-X8 ($H^+$) (registered Trade Mark) ion exchange resin, to yield crystalline 3,3-bis(hydroxymethyl)azetidine (4.35 g; 90% yield on the starting oxetane.

The above procedure may be effected using 2,2-bis(bromomethyl)-1,3-propanediol as starting material giving the same end product in similar yield. Alternatively 3,3-bis(hydroxymethyl)azetidine may be prepared by debenzylation of 3,3-bis(hydroxymethyl)-1-benzylazetidine using hydrogen over palladium on carbon catalyst in methanol.

Example 3

3,3-Bis(hydroxymethyl)-1-methylazetidine was prepared by a process similar to that of Example 2 using methylamine instead of ammonia.

Example 4

3,3-Bis(hydroxymethyl)-1-ethylazetidine was prepared by a process similar to that of Example 2 using ethylamine instead of ammonia.

Example 5

3,3-bis(hydroxymethyl)-1-isopropylazetidine was prepared by a process similar to that of Example 2 using isopropylamine instead of ammonia.

## Example 6

Preparation of 3,3-bis(hydroxymethyl)-1-benzylazetidine

(i) A mixture of 2,2-bis(bromomethyl)-1,3-propanediol (10 g, 0.038 mol), acetone (5 ml) and a catalytic quantity (0.1 g) of p-toluenesulphonic acid was heated under reflux in benzene (150 ml) until the theoretical amount of water (0.8 ml) had been collected in a Dean-Stark trap (2 hours). Evaporation of the solvent gave 5,5-bis(bromomethyl)-2,2-dimethyl-1,3-dioxane (11.5 g) in 99% yield. The purity according to gas-liquid chromatography and proton NMR was 99%, m.p. 56—58°C.

(ii) A mixture of 5,5-bis(bromomethyl)-2,2-dimethyl-1,3-dioxane (28.5 g, 0.094 mol) and sodium carbonate (12 g, 0.28 mol) in dimethylsulphoxide (50 ml) was heated to 135°C. To the mixture was added dropwise with stirring a solution of benzylamine (13.5 g, 0.126 mol) in dimethylsulphoxide (50 ml) over a period of 5 to 6 hours. When the addition had been completed the reaction mixture was stirred for 7 hours at 135°C. After cooling, water (10 ml) was added and the whole extracted with pentane (4x50 ml). The pentane extracts were washed with water (3x20 ml), dried and evaporated to leave 15.84 g of an oil. Distillation under reduced pressure gave 3,3-bis(hydroxymethyl)-1-benzylazetidine acetone acetal (13.2 g) as a colourless oil in 56.6% yield (90% pure) b.p. 135—136°C at 267 Pa (2m Hg).

(iii) The acetal from step (ii) (10 g, 0.036 mol) was heated for 2 hours at 50°C in 5% w/w sulphuric acid (25 ml), cooled to ambient temperature and made basic with 10% (w/w) sodium hydroxide. Extraction with ethyl acetate (x3) gave after drying and evaporating an off-white solid (5 g). Recrystallisation from toluene (20 ml) then gave pure 3,3-bis(hydroxymethyl)-1-benzylazetidine as white crystals in 54% yield m.p. 86—87°C.

| | | | |
|---|---|---|---|
| Analysis: Calc. | C 69.54, | H 8.27, | N 6.76 |
| Found: | C 69.25, | H 8.27, | N 6.65 |

## Example 7

Preparation of 3,3-bis(hydroxymethyl)-1-benzylazetidine

5,5-bis(bromomethyl)-2,2-dimethyl-1,3-dioxane (30.2 g, 0.1 mol) and sodium bicarbonate (24 g, 0.3 mol) in dimethylsulphoxide (50 ml) were heated to 135°C. A solution of benzylamine (10.7 g, 0.1 mol) in dimethylsulphoxide (50 ml) was then added dropwise with stirring over 1.5 hours. After addition was complete the mixture was stirred for 2.5 hours at 135°C. After cooling, dimethylsulphoxide (100 ml) and water (20 ml) were added and the solution was extracted with pentane (5x100 ml). The combined pentane extracts were washed with water (3x25 ml) and then shaken with 5% (w/w) sulphuric acid (×3) in order to extract the basic 3,3-bis(hydroxymethyl)-1-benzylazetidine acetone acetal.

The acidic aqueous layer was heated for 1 hour at 50°C and then evaporated nearly to dryness. The residue was made basic with 10% (w/w) sodium hydroxide and evaporated to dryness under reduced pressure. Ethanol was added and the insolubles filtered off. Evaporation of the ethanol left a white solid which on recrystallisation from toluene gave 3,3-bis(hydroxymethyl)-1-benzylazetidine (9.8 g, 0.047 mol) as white crystals m.p. 86.2—86.8°C in 47% yield based on 5,5-bis(bromomethyl)-2,2-dimethyl-1,3-dioxane. Unreacted 5,5-bis(bromomethyl)-2,2-dimethyl-1,3-dioxane (4.6 g, 0.015 mol) was recovered from the washed pentane extracts by washing with aqueous sodium carbonate, then with water, drying and evaporating off the pentane.

## Example 8

Preparation of azetidine-3-carboxylic acid

(i) 3,3-bis(hydroxymethyl)-1-benzylazetidine (10 g) was dissolved in 55% w/w aqueous nitric acid (100 ml) and the resulting mixture was stirred for 18 hours at 50°C. Benzoic acid precipitated out as a white solid and was filtered off. The filtrate was evaporated to dryness, water (50 ml) was added and the solution again evaporated to dryness. This latter step was repeated three times. The residue was then dissolved in water (50 ml) and the solution was extracted with diethyl ether (100 ml) to extract any remaining benzoic acid. Evaporation of the water left 7.3 g residue, which was shown by 90 MHz proton NMR to be 100% pure 1-nitrosoazetidine-3,3-dicarboxylic acid.

(ii) To the 1-nitrosoazetidine-3,3-dicarboxylic acid from step (i) (7.3 g) was added water (10 ml) and formic acid (40 ml). The resulting mixture was heated for 5 hours at 110°C. Nitrous fumes evolved. The mixture was then evaporated to dryness, yielding 4.9 g of a brown oil which was shown by high pressure liquid chromatography against standard material to contain 43.5%w of azetidine-3-carboxylic acid.

## Examples 9 and 10

The products of Example 3, 3,3-bis(hydroxymethyl)-1-methylazetidine, and of Example 4, 3,3-bis(hydroxymethyl)-1-ethylazetidine, were oxidised under the conditions of Example 8(i), and in each case the product was 1-nitrosoazetidine-3,3-dicarboxylic acid.

## Examples 11 to 13

Oxidation of 3,3-bis(hydroxymethyl)-1-benzylazetidine

3,3-bishydroxymethyl-1-benzylazetidine was oxidised with nitric acid by similar general procedures to that of Example 8(i) but with variations in concentrations and conditions. Results are given in Table 1 following.

Table 1

| Example | Scale (g) | Nitric acid concentration (w/w) | Temperature (°C) | Concentration of azetidine starting material (g/ml) | Reaction time (hours) | Product (yield, %w)* |
|---|---|---|---|---|---|---|
| 11 | 1 | 65 | 50 | 1/5 | 18 | 1-nitrosoazetidine-3,3-dicarboxylic acid (83%) |
| 12 | 5 | 55 | 50 | 1/10 | 16.5 | 1-nitrosoazetidine-3,3-dicarboxylic acid (79%) |
| 13 | 2.5 | 55 | 50 | 1/20 | 18 | 1-nitrosoazetidine-3,3-dicarboxylic acid (71%) azetidine-3,3-dicarboxylic acid (23%) |

* All yields are calculated on the basis of weights and 90MHz proton NMR results of isolated products, assuming 100% pure starting material.

Examples 14 to 16

Oxidation of 3,3-bis(hydroxymethyl)azetidine

3,3-bis(hydroxymethyl)azetidine was oxidised with nitric acid by procedures comparable with those of Example 8(i). Experimental conditions and results are given in Table 2 following.

Table 2

| Example | Nitric acid concentration (w/w) | Temperature (°C) | Concentration of azetidine starting material (g/ml) | Reaction time (hours) | Product (yield, %w)* |
|---|---|---|---|---|---|
| 14 | 55 | 50 | 1/20 | 16.5 | 1-nitrosoazetidine-3,3-dicarboxylic acid (74%) |
| 15 | 55 | 50 | 1/15 | 4 | 1-nitrosoazetidine-3,3-dicarboxylic acid (73%) |
| 16 | 55 | 50 | 1/20 | 18 | 1-nitrosoazetidine-3,3-dicarboxylic acid (63%) azetidine-3,3-dicarboxylic acid (13%) |

* All yields are calculated as for Table 1.

Examples 17 to 19

Conversion of 1-nitrosoazetidine-3,3-dicarboxylic acid

1-Nitrosoazetidine-3,3-dicarboxylic acid was subjected to acid treatment following procedures comparable with that of Example 8(ii). Experimental conditions and results are given in Table 3 following. Comparative Example A demonstrates that acid conditions are necessary for full conversion, but demonstrates that 1-nitrosoazetidine-3-carboxylic acid is an intermediate in conversion of 1-nitrosoazetidine-3,3-dicarboxylic acid to azetidine-3-carboxylic acid.

Table 3

| Example | Acid (concentration w/w) | Temperature | Reaction time (hours) | Product (yield, %w)* |
|---|---|---|---|---|
| 17 | hydrochloric, 9% w/w | reflux | 16.5 | azetidine-3-carboxylic acid hydrochloride (68%) (pale yellow solid) |
| 18 | hydrochloric, 3.6% w/w | reflux | 16.5 | azetidine-3-carboxylic acid hydrochloride (70%) (pale yellow solid) |
| 19 | formic acid, 80% v/v | reflux | 16.5 | azetidine-3-carboxylic acid (77%) (dark brown oil) |
| Comparative A | water | reflux | 16.5 | resulting solution contained mixture of 1-nitrosoazetidine-3,3-dicarboxylic acid, 1-nitrosoazetidine-3-carboxylic acid and azetidine-3-carboxylic acid in molar ratio 1:4:2 |

* All yields are calculated as for Table 1.

**Claims**

1. A process for preparing azetidine-3-carboxylic acid

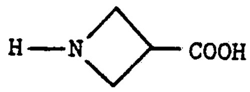

(I)

or a salt thereof, which comprises treating a compound of formula

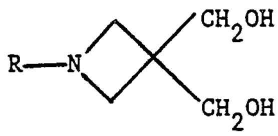

(II)

wherein R represents a hydrogen atom or a group of formula $R^1R^2CH$— wherein $R^1$ and $R^2$ are independently selected from hydrogen, alkyl and optionally substituted aryl moieties, with nitric acid and subjecting the resulting product to acid conditions at elevated temperature to produce an acid-addition salt of formula I, and thereafter, if desired, converting the acid-addition salt to the free acid or another salt of the acid of formula I.

2. A process according to claim 1 wherein R in formula II represents a hydrogen atom or a group of formula $R^1CH_2$— wherein $R^1$ represents hydrogen, a $C_{1-3}$alkyl group or a phenyl group.

3. A process according to claim 1 or 2 wherein the compound of formula II is treated with nitric acid at a temperature in the range 40°C to the reflux temperature of the reaction mixture.

4. A process according to any of Claims 1 to 3 wherein the concentration of the nitric acid used is in the range 50 to 65%w/w.

5. A process according to any of Claims 1 to 4 wherein subjecting the resulting product to acid conditions comprises treating the product with an aqueous acid selected from nitric, formic, hydrochloric and sulphuric acids.

6. A 1-nitroso-azetidine-3-carboxylic acid derivative of formula

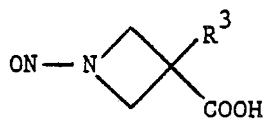

(III)

or a salt thereof wherein $R^3$ is hydrogen or a group of formula COOH.

**Patentansprüche**

1. Verfahren zur Herstellung von Azetidin-3-carbonsäure

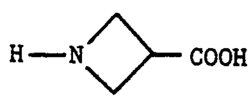

(I)

oder eines Salzes davon, welches Verfahren ein Behandeln einer Verbindung der Formel

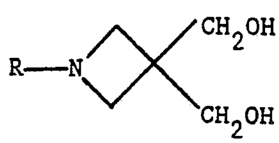

(II)

worin R ein Wasserstoffatom oder ein Gruppe der Formel $R^1R^2CH$—, worin $R^1$ und $R^2$ unabhängig voneinander unter Wasserstoff, Alkyl- und gegebenenfalls substituierten Arylresten ausgewählt sind, bedeutet, mit Salpetersäure und ein Einwirkenlassen saurer Bedingungen bei erhöhten Temperaturen auf das gebildete Produkt zur Ausbildung eines Säureadditionssalzes der Formel I und hiernach gewünschtenfalls ein Überführen des Säureadditionssalzes in die freie Säure oder in ein anderes Salz der Säure I umfaßt.

2. Verfahren nach Anspruch 1, worin R in Formel II ein Wasserstoffatom oder eine Gruppe der Formel $R^1CH_2$—, worin $R^1$ Wasserstoff, eine $C_{1-3}$Alkylgruppe oder eine Phenylgruppe bedeutet, darstellt.

3. Verfahren nach Anspruch 1 oder 2, worin eine Verbindung der Formel II mit Salpetersäure bei einer Temperatur im Bereich von 40°C bis zur Rückflußtemperatur des Reaktionsgemisches behandelt wird.

4. Verfahren nach einem der Ansprüche 1 bis 3, worin die Konzentration der eingesetzten Salpetersäure im Bereich von 50 bis 60% Gewicht/Gewicht liegt.

5. Verfahren nach einem der Ansprüche 1 bis 4, worin das Einwirkenlassen sauer Bedingungen auf das erhaltene Produkt ein Behandeln des Produktes mit einer wäßrigen Säure, ausgewählt unter Salpetersäure, Ameisensäure, Salzsäure und Schwefelsäure, umfaßt.

6. 1-Nitrosoazetidin-3-carbonsäurederivat mit der Formel

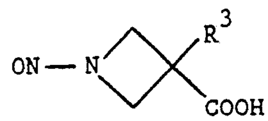

(III)

oder ein Salz hievon, worin $R^3$ Wasserstoff oder eine Gruppe der Formel COOH bedeutet.

**Revendications**

1. Un procédé de préparation d'acides azétidine-3-carboxylique

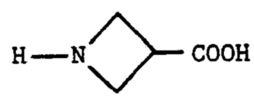

(I)

ou d'un sel de cet acide, qui comprend le traitement d'un composé de formule

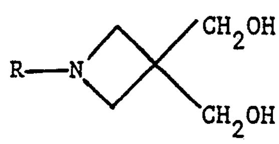

(II)

où R représente un atome d'hydrogène ou un groupe de formule $R^1R^2CH$— où $R^1$ et $R^2$ sont choisis indépendamment parmi l'hydrogène, des portions alcoyle et des portions aryle éventuellement substituées, par l'acide nitrique et l'exposition du produit résultant à des conditions acides à température élevée pour produire un sel d'addition d'acide de formule I et ensuite, si on le désire, la conversion du sel d'addition d'acide en l'acide libre ou en un autre sel de l'acide de formule I.

2. Un procédé selon la revendication 1, dans lequel R dans la formule II représente un atome d'hydrogène ou un groupe de formule $R^1CH_2$— où $R^1$ représente un atome d'hydrogène, un groupe alcoyle en $C_{1-3}$ ou un groupe phényle.

3. Un procédé selon la revendication 1 ou 2, dans lequel le composé de formule II est traité par l'acide nitrique à une température comprise entre 40°C et la température de reflux du mélange réactionnel.

4. Un procédé selon l'une quelconque des revendications 1 à 3, dans lequel la concentration de l'acide nitrique utilisé est comprise entre 50 et 65% en poids/poids.

5. Un procédé selon l'une quelconque des revendications 1 à 4, dans lequel l'exposition du produit résultant à des conditions acides comprend le traitement du produit par un acide aqueux choisi parmi les acides nitrique, formique, chlorhydrique et sulfurique.

6. Un dérivé d'acide 1-nitroso-azétidine-3-carboxylique de formule

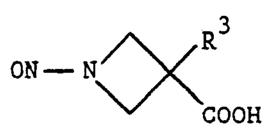

(III)

ou un sel d'un tel composé, où $R^3$ est de l'hydrogène ou un groupe de formule COOH.